# EUROPÄISCHE PATENTSCHRIFT

(11) **EP 1 656 964 B1**
(45) Veröffentlichungstag und Bekanntmachung des Hinweises auf die Patenterteilung: **14.01.2015**
(21) Anmeldenummer: 06003738.9
(22) Anmeldetag: 11.12.2002
(51) Int. Cl.: A61M 25/00, A61M 25/06

(54) **Katheter**
Catheter
Cathéter

(43) Veröffentlichungstag der Anmeldung: 17.05.2006
(62) Teilanmeldung aus: 02027767.9
(73) Patentinhaber: Abbott Laboratories Vascular Enterprises Limited, Dublin 2 (IE)
(72) Erfinder: Schneider, Ralph, Irvine, CA 92603 (DE); Hartwig, Judith, 72415 Grosselfingen (DE); Nielsen, Stevan, 72108 Rottenburg (DE)
(74) Vertreter: Schmitz, Hans-Werner

(56) Entgegenhaltungen:
- EP-A- 0 792 655
- EP-A- 0 937 480
- WO-A-92/18193
- WO-A-96/16690
- DE-A- 3 907 549
- FR-A- 1 290 933
- US-A- 5 741 429
- US-B1- 6 387 075

## Beschreibung

Die Erfindung betrifft einen Katheter gemäß dem Oberbegriff des Anspruches 1.

Ein gattungsgemäßer Katheter, der beispielsweise aus dem US-Patent 5,569,200 bekannt ist, weist einen Katheterschaft auf, der üblicherweise als "Hypotube" bezeichnet wird.

Am proximalen Ende dieses Katheterschaftes ist ein Anschlussstück, vorzugsweise in Form eines Luer-Anschlussstückes, vorgesehen, das zum Anschluss einer Druckvorrichtung dient, über die durch im Anschlussstück vorgesehene Kanäle die Druckflüssigkeit zum Expandieren des Ballons am distalen Ende des Katheterschaftes eingeleitet wird.

Im Rahmen der Erfindung durchgeführte Untersuchungen haben gezeigt, dass die Übergangsstelle zwischen dem Hypotube und dem Anschlussstück sehr knickempfindlich ist. Hierbei kann der Benutzer den Katheterschaft abknicken, wenn er ihn aus der Verpackung und der Schutzhülle entnimmt oder wenn er die Druckvorrichtung anschließt. Die im Rahmen der Erfindung durchgeführten Untersuchungen haben hierzu ergeben, dass zumindest einer der Gründe für dieses unerwünschte Abknicken die Tatsache ist, dass der Katheterschaft einen sehr geringen Durchmesser aufweist, sodass der Benutzer den Katheterschaft beim Entnehmen aus der Verpackung oder beim Anschließen der Druckvorrichtung nicht ausreichend gut fühlt und ihn so unbeabsichtigter Weise abknicken kann. Das Abknicken ist deshalb besonders kritisch, da es zu einem Verschluss des Inflationslumens führen kann, der den Katheter funktionsunfähig macht.

Es ist daher Aufgabe der vorliegenden Erfindung, einen Katheter der im Oberbegriff des Anspruches 1 angegebenen Art zu schaffen, dessen Handlingeigenschaften verbessert sind und der insbesondere nicht knickanfällig ist.

Die Lösung dieser Aufgabe erfolgt durch die Merkmale des Anspruches 1.

Im gattungsgemäßen Stand der Technik gemäß dem US-Patent 5,569,200 ist die Knickempfindlichkeit zwar auch angesprochen und es wird das Vorsehen von Schlitzen im Katheterschaft als eine mögliche Lösung dieses Problems angegeben, jedoch sind diese Schlitze am distalen Ende vorgesehen, an welcher Stelle keine Kräfte quer zur Katheterlängsachse wirken, da nur Kräfte in Richtung der Katheterlängsachse aufgebracht werden. Insofern können diese Schlitze das eingangs erläuterte Problem nicht beseitigen, da beim gattungsgemäßen Katheter weiterhin die Gefahr des Abknickens bzw. sogar Abbrechens beim Aufbringen externer Kräfte, wie zuvor beschrieben, besteht.

Demgegenüber wird durch das bewusste Vorsehen eines Biegeabschnittes am proximalen Ende des Katheterschaftes erfindungsgemäß auf verblüffend einfache und nicht vorhersehbare Art und Weise das der Erfindung zugrundeliegende technische Problem gelöst, da das bewusste Vorsehen eines Biegeabschnittes gerade im kritischen Bereich des Katheterschaftes, der beim gattungsgemäßen Katheter besonders knickempfindlich ist, eine höhere Flexibilität schafft, die zwar ein gewolltes Abbiegen möglich macht, dadurch jedoch das unerwünschte Abknicken oder sogar Abbrechen sicher verhindert wird, da eine plastische Deformation des Katheterschaftes verhindert wird.

Ferner ist der Biegeabschnitt aus einer Mehrzahl von Kugeln ausgebildet, die auf das proximale Ende des Katheterschaftes aufgebracht sind. Diese Kugeln können entweder mechanisch auf dem Katheterschaft (Hypotube) aufgebracht werden, oder durch Kleben, Schweißen oder einstückig angeformt sein. Ferner ist es möglich, zur Lagefixierung über die Kugeln einen Schrumpfschlauch aufzubringen.

Die erfindungsgemäße Aufgabe wird erfindungsgemäss durch ein mechanisches Beschränken des maximalen Biegemaßes und durch ein Umwandeln des Biegens in einen ausreichend großen Radius erreicht, was ebenfalls die plastische Deformation des Kunststoffmaßes verhindert oder zumindest auf ein erträgliches Maß reduziert.

Dabei können die auftretenden Kräfte, die größer sind als ein möglicher maximaler Biegewinkel, in eine Längsspannung auf den Katheterschaft umgewandelt werden, welcher dieser aufgrund seiner Materialeigenschaften wesentlich besser widerstehen kann, als Biegekräfte.

Aus der US 6,387,075 ist ein weiterer Katheter bekannt, der eine distal des gewebten Katheterschafts ins Hypotube eingeschnittene Spirale aufweist. Diese Spirale soll jedoch lediglich zur Verbesserung der Flexibilität des Katheterschafts im distal gelegenen Bereich des Katheters dienen. Die Spirale kann daher entsprechend der zuvor genannten US 5,569,200 die Gefahr des Abknickens des Katheters hinter dem Anschlussstück nicht vermeiden.

In der US 6,387,075 wird ferner beschrieben, dass zur Vermeidung des Abknickens des Katheters hinter dem Anschlussstück der proximale Katheterschaft auf einer Länge von etwa 6 bis 12 inch mit einem Polymer verstärkt oder durch eine Armierung stabilisiert wird. Mit dieser Verstärkung wird jedoch lediglich die Knickstelle in distale Richtung gegen das Ende der Verstärkung verschoben und darüberhinaus verringert die Polymer-Verstärkung die zur Verfügung stehende benutzbare Länge des Katheters und ergibt somit eine unerwünschte Verlängerung des Katheterschaftes.

Schließlich wird bei einer weiteren Ausführungsform zur Verstärkung des proximalen Katheterschaftes in der US 6,387,075 ein Kathether beschrieben, dessen proximaler Schaft statt aus einem Hypotube aus einem zwischen einem inneren und einem äußeren Polymer-Tube gelegenen Gewebe besteht. Diese Ausführungsform umgeht das Problem des Abknickens des Katheters durch den Einbau eines Katheterabschnittes, der nicht aus dem Hypotube, sondern aus neuen, zusätzlichen Schaft-Komponenten besteht. Hierbei ergeben sich jedoch große Nachteile durch die Verringerung der pushability and die aufwändige und teure Herstellung des Katheters.

Die Unteransprüche haben vorteilhafte Weiterbildungen der Erfindung zu Inhalt.

Weitere Einzelheiten, Merkmale und Vorteile der Erfindung ergeben sich aus nachfolgender Beschreibung von Ausführungsbeispielen anhand der Zeichnung.

Es zeigt:
- Fig. 1: eine schematisch stark vereinfachte Darstellung eines erfindungsgemäßen Katheters,
- Fig. 2: eine der Fig. 1 entsprechende Darstellung einer dritten grundsätzlich möglichen Ausführungsform des erfindungsgemäßen Katheters, und
- Fig. 3-5: Darstellungen von Teilen des in Fig. 2 wiedergegebenen Katheters zur Erläuterung der Wirkungsweise dieser Ausführungsform.

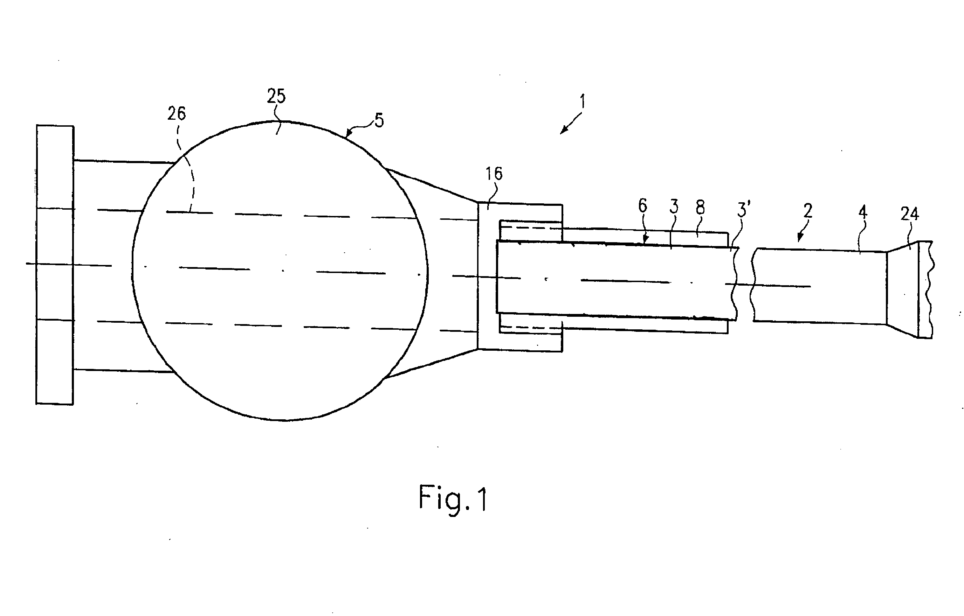

In Fig. 1 ist ein erfindungsgemäßer Katheter 1 dargestellt, der einen Katheterschaft 2 aufweist. Der Katheterschaft 2 ist mit einem proximalen Ende 3 und einem distalen Ende 4 versehen, die in Fig. 1 stark verkürzt dargestellt sind. Am distalen Ende 4 ist ein schematisch nur angedeuteter Ballon 24 befestigt. Ferner weist der Katheter 1 ein Anschlussstück 5 auf, das üblicherweise als Luer-Anschlussstück ausgebildet ist. Dieses Anschlussstück 5 ist am proximalen Ende 3 des Katheterschaftes 2 befestigt und kann ein üblich ausgebildetes Gehäuse 25 mit einem Durchtrittskanal 26 aufweisen, der mit dem Katheterschaft 2 in Strömungsverbindung steht. Dieses Anschlussstück 5 wird mit einer Druckvorrichtung (Inflationdevice) verbunden, um den Ballon 24 aufzuweiten.

Das proximale Ende 3 des Katheterschaftes 2 des erfindungsgemäßen Katheters 1 ist mit einem Biegeabschnitt 6 versehen, dessen Biegsamkeit größer ist, als diejenige des sich an das proximale Ende 3 anschließenden Abschnittes 3' des Katheterschaftes 2. Dieser Biegeabschnitt 6 verhindert ein ungewolltes Abknicken auf die eingangs erläuterte Art und Weise.

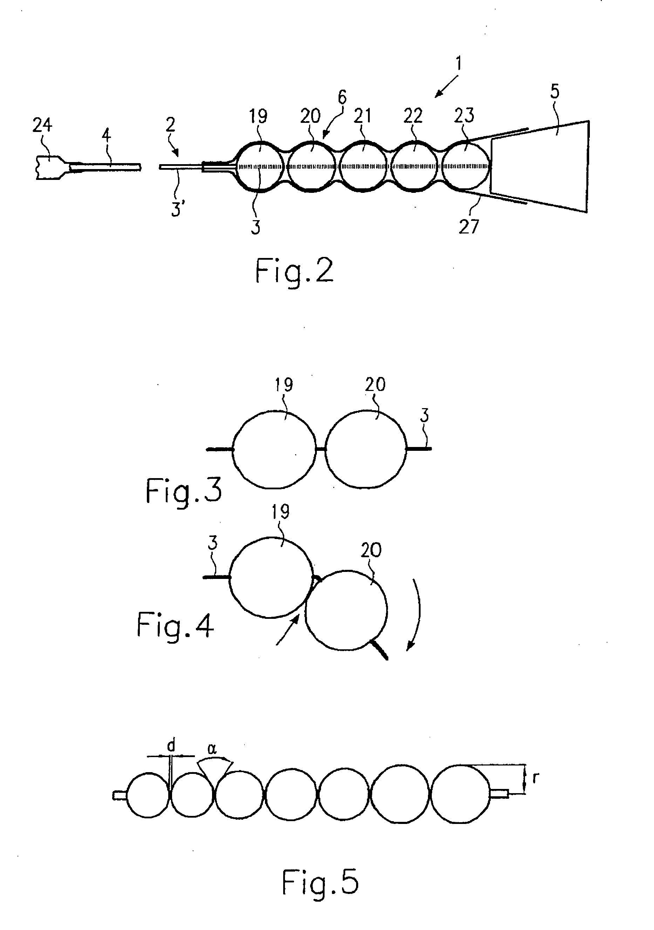

In Fig. 2 ist der erfindungsgemäße Katheter 1 im Detail nochmals dargestellt, bei der wiederum sämtliche Teile, die der Ausführungsform gemäß Fig. 1 entsprechen, mit denselben Bezugsziffern versehen sind.

Bei dieser Ausführungsform weist der Biegeabschnitt 6 eine Mehrzahl von auf das proximale Ende 3 aufgebrachte Kugeln 19 bis 23 auf. Diese Kugeln können durch unterschiedliche Befestigungsmöglichkeiten, die eingangs erläutert wurden, am Katheterschaft 2 fixiert werden. Bei der in Fig. 6 dargestellten Ausführungsform ist hierfür ein Schrumpfschlauch 27 vorgesehen, der an einem Ende mit dem Katheterschaft 2 verbunden ist und am anderen Ende am Anschlussstück 5 fixiert ist.

Die Fig. 3 und 4 erläutern die Wirkungsweise dieser Ausführungsform anhand zweier Kugeln 19 und 20. Hierbei wird deutlich, dass die Kugeln in ihrem Abstand d und in ihrem Radius r (siehe Fig. 5) so bemessen werden können, dass ein maximal tolerabler Biegewinkel α (siehe Fig. 5) möglich gemacht wird, der keine plastische Deformation und damit ein Abknicken oder sogar Abbrechen des Katheterschaftes zur Folge hat.

Auch Katheter, die keinen Ballon aufweisen, sind als erfindungsgemäße Katheter zu verstehen.

## Patentansprüche

1. Katheter (1)
- mit einem Katheterschaft (2), der ein proximales Ende (3) und ein distales Ende (4) aufweist, an dem vorzugsweise ein Ballon (24) befestigt ist, und
- mit einem Anschlussstück (5), vorzugsweise einem Luer-Anschlussstück, das am proximalen Ende (3) des Katheterschaftes (2) angeordnet ist,
**dadurch gekennzeichnet,**
- **dass** das proximale Ende (3) des Katheterschaftes (2) mit einem Biegeabschnitt (6) versehen ist, und
- **dass** der Biegeabschnitt (6) eine Mehrzahl von Kugeln (19 bis 23) aufweist, die auf dem proximalen Ende (3) des Katheterschaftes (2) aufgebracht sind.

2. Katheter nach Anspruch 1, **dadurch gekennzeichnet, dass** der Biegeabschnitt (6) eine Biegsamkeit aufweist, die größer ist als diejenige des sich an das proximale Ende (3) anschließenden Abschnitts (3') des Katheterschaftes (2).

3. Katheter nach Anspruch 1, **dadurch gekennzeichnet, dass** der Biegeabschnitt (6) als weichgeglühter Materialabschnitt des Katheterschaftes (2) ausgebildet ist.

## Claims

1. A catheter (1) comprising
- a catheter shaft (2) including a proximal end (3) and a distal end (4) to which a balloon (24) is preferably fastened, and
- a fitting (5), preferably a luer fitting, arranged at the proximal end (3) of the catheter shaft (2),
**characterized in**
- **that** the proximal end (3) of the catheter shaft (2) is provided with a bending section (6), and
- **that** the bending section (6) includes a plurality of balls (19 to 23) which are mounted on the proximal end (3) of the catheter shaft (2).

2. The catheter according to claim 1, **characterized in that** the bending section (6) has a flexibility greater than that of the section (3') of the catheter shaft (2) joining the proximal end (3).

3. The catheter according to claim 1, **characterized in that** the bending section (6) is formed as a soft-annealed material section of the catheter shaft (2).

## Revendications

1. Cathéter (1)
- comprenant une tige de cathéter (2), laquelle présente une extrémité proximale (3) et une extrémité distale (4), au niveau de laquelle un ballon (24), de préférence, est fixé, et
- comprenant une pièce de raccordement (5), de préférence une pièce de raccordement de type Luer, laquelle est agencée au niveau de l'extrémité proximale (3) de la tige de cathéter (2),
**caractérisé en ce que**
- l'extrémité proximale (3) de la tige de cathéter (2) est munie d'une partie souple (6), et
- la partie souple (6) présente une pluralité de boules (19 à 23), lesquelles sont disposées sur l'extrémité proximale (3) de la tige de cathéter (2).

2. Cathéter selon la revendication 1, **caractérisé en ce que** la partie souple (6) présente une certaine souplesse, qui est plus importante que celle de la partie (3') se situant juste après l'extrémité proximale (3) de la tige de cathéter (2).

3. Cathéter selon la revendication 1, **caractérisé en ce que** la partie souple (6) est préparée comme partie de matériau recuite de la tige de cathéter (2).
